Europäisches Patentamt

European Patent Office

Office européen des brevets

(11) Publication number: **0 325 489 B1**

## EUROPEAN PATENT SPECIFICATION

(45) Date of publication of patent specification: **19.01.94**

(51) Int. Cl.5: **C12N 5/08**, C12P 21/08, G01N 33/577, A61K 39/395

(21) Application number: **89300570.2**

(22) Date of filing: **20.01.89**

The file contains technical information submitted after the application was filed and not included in this specification

(54) **Leu 23: Monoclonal antibody for monitoring leukocyte activation.**

(30) Priority: **21.01.88 US 146745**

(43) Date of publication of application:
**26.07.89 Bulletin  89/30**

(45) Publication of the grant of the patent:
**19.01.94 Bulletin  94/03**

(84) Designated Contracting States:
**AT BE CH DE ES FR GB GR IT LI LU NL SE**

(56) References cited:
**WO-A-85/02188
US-A- 4 599 304
US-A- 4 607 007**

**CHEMICAL ABSTRACTS ,vol.109,no.1,july 4, 1988,Columbus, Ohio, USA L. L. LANIER et al. "Interleukin 2 activation of natural killer cells rapidly induces the expression and phosphorylation of the Leu-23 activation antigen" page 484, right column, Abstract-no. 5 026s & J. Exp. Med. 1988, 167(5), 1572-85**

**CHEMICAL ABSTRACTS, vol. 109, no. 23 December 5, 1988 Columbus, Ohio, USA R.**

**TESTI et al. "Constitutive expression of a phosphorylated activation antigen (Leu 23) by CD3 bright human thimocytes" page 507, right column, Abstract-no. 209 311r & J. Immunol. 1988, 141(8), 2557-63**

(73) Proprietor: **Becton Dickinson and Company One Becton Drive Franklin Lakes New Jersey 07417-1880(US)**

(72) Inventor: **Phillips, Joseph H.
305 Almaden Way
San Mateo, California(US)**
Inventor: **Lanier, Lewis L.
1528 Frontero Avenue
Los Altos, California(US)**

(74) Representative: **Ruffles, Graham Keith et al
MARKS & CLERK
57-60 Lincoln's Inn Fields
London WC2A 3LS (GB)**

EP 0 325 489 B1

**Description**

Field of the Invention

This invention relates to a monoclonal antibody (MAb) useful in monitoring leukocyte activation, and more particularly, related to a monoclonal antibody that will bind an antigen that appears on natural killer (NK) cells, on a subset of low bouyant density (LBD) T lymphocytes rapidly after such cells are activated with Interleukin-2 (IL-2) and on certain T lymphocytes after stimulation of the T cell antigen receptor complex.

Background of the Invention

IL-2 potentiates the growth and cytotoxic function of both T lymphocytes and NK cells. Upon exposure to IL-2 (or recombinant IL-2), resting NK cells isolated from peripheral blood will demonstrate enhanced cytotoxic activity within 4 hours after activation. This cytotoxic activity is maximal after 18 hours.

Most other peripheral blood leukocytes, by contrast, do not respond to IL-2 alone. Additional signals, such as IL-1 or protein kinase C, are required to induce growth. A subset of LBD T cells, however, do respond to IL-2 alone, and thus, appear to be in a "primed" state.

Once activated, leukocytes express a variety of new cell surface antigens. NK cells, for example, will express transferrin receptor, HLA-DR and the CD25 IL-2 receptor after IL-2 activation. These antigens, however, are not expressed on a majority of cells until well after the 4-18 hour time period when NK cells are the most cytotoxic. Thus, there is no direct correlation between NK activation and cytotoxic function and the expression of these antigens. As a result, there are no means to measure the effectiveness of activation until after maximal cytotoxicity has passed.

Measuring the effectiveness of activation of NK cells and hence their effectiveness as cytotoxic agents, for example, is important in the treatment of certain diseases. Recently, Rosenberg et al. in N. Eng. J. Med., 164:814 (1985), and Rosenberg in U.S. Pat. No. 4,690,915 proposed a method of cancer therapy that involves the combined in vivo use of IL-2 with administration of the patient's autologous lymphocytes that have been activated in vitro with IL-2. It would be useful to measure activation of the lymphocytes prior to administration to insure maximal therapeutic benefit.

In addition to the activation of leukocytes by IL-2, it is possible to stimulate T cells, for example, by interaction with the T cell antigen receptor complex which comprises alpha and beta chains in the antigen receptor and which appear and complex with several other proteins denominated as the CD3+ complex. The T cell antigen receptor is responsible for recognition of foreign antigens (e.g., alloantigen and virus) and causes an activated T cell to undergo certain transformations which may effect immune responses. It further would be useful to have a marker to identify cells that had been stimulated via the T cell antigen receptor complex.

Hara et al., Human T Cell Activation: III, Rapid Induction of a Phosphorylated 28 kD / 32 kD Disulfide-linked Early Activation Antigen (EA-1) by 12-0-tetradecanoyl Phorbol-13-Acetate, Mitogens and Antigens, J. Exp. Med., 164:1988 (1986), and Cosulich et al., Functional Characterization of an Antigen (MLR3) Involved in an Early Step of T-Cell Activation, PNAS, 84:4205 (1987), recently have described cell surface antigens that are expressed on T cells shortly after activation. These antigens, EA-1 and MLR3 respectively, are glycoproteins having major components of 28 kD and 32 kD. EA-1 and MLR3 are not HLA class II antigens and an MLR3 MAb will block IL-1 binding. These antigens appear on activated T cells within 18 hours and continue to appear as late as 48 hours after activation.

Although these antigens may be useful in detecting activation, the present invention provides an alternative means to detect activation of leukocytes, and in particular NK cells and a subset of T cells.

Summary of the Invention

In accordance with the present invention, there is provided a monoclonal antibody which interacts with a cellular antigen recognised by the monoclonal antibody produced by the hybridoma designated as ATCC No. HB-9627.

The invention further provides a hybridoma which produces a monoclonal antibody, said monoclonal antibody interacting with the cellular antigen which is recognised by the monoclonal antibody produced by the hybridoma designated as HB-9627.

A mouse hybridoma L78 was formed between cells from the popliteal lymph nodes of Balb/c mice immunized with a CD8+ alloantigen-directed cytotoxic T lymphocyte (CTL) cell line and the SP2/0 Ag 14 murine myeloma cell line. Fused cells were cultured and selected in an azaserine-hypoxanthine media. Clone LR3-8H3 was selected and renamed L78. L78 has been deposited with the American Type Culture Collection, Rockville, Maryland under the terms of the Budapest Treaty with the accession number ATCC HB-9627.

A mouse hybridoma L-184 was formed between splenocytes from Balb/c mice immunized with rIL-2 activated NK cells and the SP2/O Ag 14 murine myeloma cell line. Clone E14/A97 was isolated and was renamed L184.

The monoclonals produced by L78 and L184 have been designated as Anti-Leu23, and have an $IgG_1$ isotype. Thus, in this specification, the designation "Leu-23" indicates in particular the cellular antigen recognised by the monoclonal antibody produced by the hybridoma designated as ATCC No. HB-9627, and analogously "Anti-Leu 23" is antibody recognising this antigen.

Anti-Leu 23 recognizes a cell surface antigen on activated and antigen stimulated leukocytes. On rIL-2 activated NK cells, the antigen, Leu 23, is expressed within 4 hours after activation and continues to be expressed as late as 72 hours after activation. Leu 23 is a disulfide-linked homodimer composed of 24 kD subunits with at least two N-linked carbohydrates. Leu 23 is both induced and phosphorylated by an IL-2 dependent process.

Because the appearance of Leu 23 on NK cells correlates with the development of cytotoxicity and because the appearance of Leu 23 on certain T cells correlates with stimulation of the T cell antigen receptor complex, Anti-Leu 23 is useful in monitoring the activation or stimulation of leukocytes.

## Brief Description of the Drawings

Fig. 1A is an autoradiograph of Anti-Leu 23 immunoprecipitates of $^{125}$I-labeled, rIL-2 activated NK cells, thymocytes and peripheral blood T lymphocytes in 2.3% sodium dodecyl sulfate (SDS) with or without 5% 2-mercaptoethanol (2-ME) and separated by polyacrylimide gel electrophoresis (PAGE) using 10% polyacrylimide;

Fig. 1B is an autoradiograph of an Anti-Leu 23 immunoprecipitate of $^{125}$I-labeled, IL-2 activated thymocytes from Fig. 1A separated in two dimensions by SDS-PAGE analysis where the first dimension was non-reducing and electrophoresed on a 7.5% polyacrylimide and where the second dimension was reducing and electrophoresed on 10% polyacrylimide;

Fig. 2 is an autoradiograph film of an Anti-Leu 23 immunoprecipitate of $^{125}$I-labeled, rIL-2 activated thymocytes from Fig. 1A wherein the immunoprecipitate was deglycosylated using endo-B-N-acetylglucosaminidase F (endo F) and separated by SDS-PAGE analysis on 10% polyacrylimide;

Fig. 3 comprises several histograms of fluorescence for LBD, HBD and NK cells activated with 500 U/ml of rIL-2 measured at 0, 2, 6 and 18 hours after activation, as detected by flow cytometry wherein activated cells were variously labeled with: fluorescein isothiocyanate (FITC) conjugated IgG and phycoerythrin (PE) conjugated anti-IgG as control antibodies; Anti-Leu 11 (FITC) and Anti-Leu 23 (PE) for NK cells (CD16$^+$); and Anti-Leu 4 (FITC) and Anti-Leu 23 (PE) for LBD and HBD cells (CD3$^+$);

Fig. 4 comprises several histograms of fluorescence for LBD cells cultured in 500 U/ml rIL-2 and, after 18 hours, variously labeled with: the controls from Fig. 3; Anti-Leu (FITC) and Anti-Leu 11 (PE) for CD16 antigen; Anti-Leu 11 (FITC) and Anti-HLA-DR (PE) for HLA-DR antigen; and Anti-Leu 11 (FITC) and Anti-IL-2R (PE) for IL-2 receptor antigen;

Fig. 5 comprises several histograms of fluorescence for LBD cells cultured in various amounts of IL-2, and, after 18 hours, labeled with the controls of Fig. 3 and Anti-Leu 11 (FITC) and Anti-Leu 23 (PE);

Fig. 6A comprises two histograms of fluorescence for LBD cells cultured in 6 U/ml of 12 U/ml rIL-2, and after 18 hours, labeled with Anti-Leu 23 (FITC) and Anti-Leu 11(PE);

Fig. 6B comprises two plots of percent cytotoxicity versus effector to target cell ratios for (unsorted LBD, CD16$^+$/Leu 23$^-$ and CD16$^+$/Leu 23$^+$ cells isolated and sorted from Fig. 6A by FACS440™ flow cytometry instrumentation as tested against $^{51}$Cr-labeled Colo-205 carcinoma target cells;

Fig. 7 comprises autoradiographs of Leu 23 and control immunoprecipitates of IL-2 activated LBD cells labeled with $^{32}$[P]-orthophosphate as analyzed by two dimensional SDS-PAGE on 10% polyacrylimide as described in Fig. 1B; and

Fig. 8 comprises two histograms of fluorescence for a leukemia cell line (Jurkat) that had been stimulated overnight with (a) an irrelevant control MAb or with (b) an anti-CD3 antibody (Anti-Leu 4) and then labeled with Anti-Leu 23 (FITC) or with an irrelevant control MAb.

Detailed Description of the Invention

The L78 hybridoma and Anti-Leu 23 MAb produced thereby were prepared as follows. $5\times10^5$ cells of a CD8$^+$ alloantigen-directed cytotoxic lymphocyte line (ElxPGF/JY, by Dr. Betty Evans, Becton Dickinson Immunocytometry Systems) were injected into the foot-pads of Balb/c mice on days 0, 4, 7,. 11, 14 and 18.

On day 19, mice were sacrificed and the popliteal lymph nodes were excised. Cells then were fused with the continuous plasmacytoma fusion partner SP2/0 Ag 14, Shulman et al., Nature, 276:269 (1978) in 35% polyethylene glycol. Similar procedures and preparations of spleen cells and fusion have been previously described in U.S. Pat. Nos. 4,172,124 and 4,196,265 which are incorporated herein by reference.

Viable cells resulting from the fusion process were plated in 96 well microculture plates (B-D Falcon) in Dulbecco's Modified Eagle's Medium (DMEM, GIBCO) containing 20% fetal calf serum (KC Biologicals). A solution of 15mM HEPES (GIBCO) and azaserine-hypoxanthine (2 ug/ml and $10^{-4}$ M final concentration respectively) was added to each well as a selection media according to the procedures of Buck et al., Production of Human Monoclonal Antibodies, in Monoclonal Antibodies and Functional Cell Lines, Plenum Publishing Corp. (Kennett et al., eds) (1984). Hybridomas grown in this medium may be separated from unfused myeloma cells and spleen cells which die shortly after fusion. Incubation continued for 7-10 days or until visable colonies appeared.

Supernatants from those wells in which cells grew were screened initially by Pandex immunoassay (Pandex Laboratories, Inc.) using CTL as the positive selection. $5\times10^5$ CTL were added to each well of a 96 well Pandex plate. To each well was added 25-50ul of supernatant. After 30 minutes, cells were washed in 0.15M phosphate buffered saline (PBS). Goat anti-mouse Ig antibody coupled to fluorescein isothiocyanate then was added to each well. The results from the immunoassay were read using Pandex instrumentation.

Cells from those wells whose supernatants tested positive were replated in 24 well microculture plates and grown in DMEM without selection media. Supernatants from each of these wells were then rescreened by adding supernatant to wells containing either rIL-2 (Cetus Corp.) activated T lymphocytes or resting peripheral blood leukocytes. Goat anti-mouse Ig(FITC) was added to each well. Stained cells from these wells were subjected to analysis by flow cytometry instrumentation (FACS440™).

Stained cells were examined for forward and right angle light scatter and fluorescence. A gate then was set for lymphocyte populations. From this analysis, clone LR3-8H3 was selected. It has been deposited with ATCC under the terms of the Budapest Treaty as accession number HB-9627. The hybridoma has been designated as L78.

L78, however, is not the sole hybridoma that will produce a MAb capable of binding Leu 23. L184 is a hybridoma which has been deposited in the laboratory of Dr. Joe Phillips (Becton Dickinson Immunocytometry Systems) that produces an Anti-Leu 23 MAb.

L184 was developed by immunizing Balb/c mice with Leu 11$^+$ sorted NK cells that had been previously activated for 1 week in rIL-2. The CTL cell line and NK cells used herein as immunogens comprise activated lymphocytes. Mice were immunized with the activated NK cells in accordance with the following schedule: $2\times10^6$ NK cells I/P on days 0, 28, 35, 42 and 49; and $2\times10^6$ NK cells I/V on days 50, 51, 52 and 53.

On day 54, spleens were excised and splenocytes were fused with the SP2/0 Ag 14 cell line as above. Selection procedures were similarly followed and clone E14/A97 ultimately was selected. Clone E14/A97 was renamed L184.

The monoclonal antibodies produced by the L78 and L184 hybridomas have been designated as Anti-Leu 23. They have an IgG$_1$ isotype. They are further characterized by the antigen with which they react.

Anti-Leu 23 reacts with essentially all IL-2 activated CD16$^+$ NK cells. Similarly, Anti-Leu 23 reacts with most IL-2 dependent CD8$^+$ CTL cell lines, CD4$^+$ tetanus-specific helper T cell clones, IL-2 dependent thymocyte cultures and mitogen activated T lymphocytes. Binding of Anti-Leu 23 to each of these cell types was assessed by direct or indirect immunofluorescence using FITC or PE labeled Anti-Leu 23 and a complementary MAb for each other cell type. These MAbs (e.g., Anti-Leu 11 (CD16), Anti-Leu 4 (CD3) and Anti-Leu 12 (CD19)) and others referred to herein are commercially available from Becton Dickinson Immunocytometry Systems, unless otherwise noted. The labeled cells were analyzed by flow cytometry on FACS $_{440}$™ instrumentation which included a Consort 40 Data Analysis System (both available from Becton Dickinson Immunocytometry Systems). The results from these analyses are presented in TABLE I.

4

## TABLE I

| Cell Type | Reactivity |
|---|---|
| Normal peripheral blood | |
|     T lymphocytes ($CD3^+$) | 0-5% positive* |
|     B lymphocytes ($CD19^+$) | 0-5% positive* |
|     NK lymphocytes ($CD16^+$) | 0-15% positive* |
|     Monocytes | weakly positive |
|     Granulocytes | 5% positive |
| Normal Thymocytes | 30% positive |
| Normal Splenocytes | 10% positive |
| IL-2 Dependent Cell Lines | |
|     $CD8^+$ Alloantigen-Directed CTL cell lines | 4/4 positive** |
|     $CD4^+$ Tetanus-toxoid helper T clones | 5/5 positive** |
|     Thymocyte cell lines | 4/4 positive** |
|     NK cell lines | 5/5 positive** |
| PHA-activated T lymphoblasts (3 days) | 50% positive |

* Based on analysis of 10 normal, adult blood donors.

** Indicates that at least 50% of cells within the cell line expressed detectable amounts of Leu 23 antigen.

While Leu 23 is prevalent on activated leukocytes, it is not preferentially expressed on proliferating cells. Several cultured T leukemia cell lines (e.g., HPB-ALL, PEER and CEM) fail to express Leu 23. Similarly, the EBV transformed B lymphoblastoid cell line, CCRF-SB, does not express Leu 23. Thus, Leu 23 is not a marker for proliferation per se, but is distributed on activated leukocytes.

Biochemical analysis of the Leu 23 antigen was conducted to demonstrate that the different cell types expressed the same antigen. Mononuclear cells were isolated from normal peripheral blood using Ficoll/Hypaque (Pharmacia). After adherence to plastic petri dishes (B-D Falcon) and passage through nylon wool to remove monocytes and B lymphocytes respectively, the remaining cells were fractionated by centrifugation on discontinuous Percoll (Pharmacia) gradients in phosphate buffered saline (PBS). The LBD fraction contains NK cells ($CD16^+$) and T lymphocytes ($CD3^+$). The high bouyant density (HBD) fraction contained resting T lymphocytes. Thymocytes were obtained from children undergoing cardiac surgery.

Thymocytes, T lymphocytes and NK cells were cultured for two weeks in medium containing rIL-2. Cultured NK cells, T cells and thymocytes were labeled with $^{125}I$ (Amersham Corp.) by the glucose oxidase/lactoperoxidase method of Keski-Oja et al., Biochem. Biophys. Res. Comm., 74:699 (1977). Cells then were solubilized in a lysis buffer (50 mM Tris-HCl, 150mM NaCl, 0.02% sodium azide, pH 8.0) containing 5mM 3[3-cholamidopropyl-dimethylammonio]-1-propane sulfate (CHAPS) detergent, 20 KI units/ml aprotinin (Sigma) and 1 mM phenyl-methane-sulfonyl fluoride (PMSF) (Sigma). Unincorporated $^{125}I$ was removed using Dowex 1x8-400 ion exchange resin (Sigma), and lysates were pre-cleared with formalin-fixed Staphylococcus aureus bearing Protein A (Pansorbin™, Calbiochem-Behring Corp.) coated with rabbit anti-mouse Ig serum (Pel-Frez Biologicals) or Anti-Leu 23. Immune complexes were eluted in sample buffer containing 2.3% SDS with or without 5% 2-ME. One or two dimensional electrophoresis then was performed.

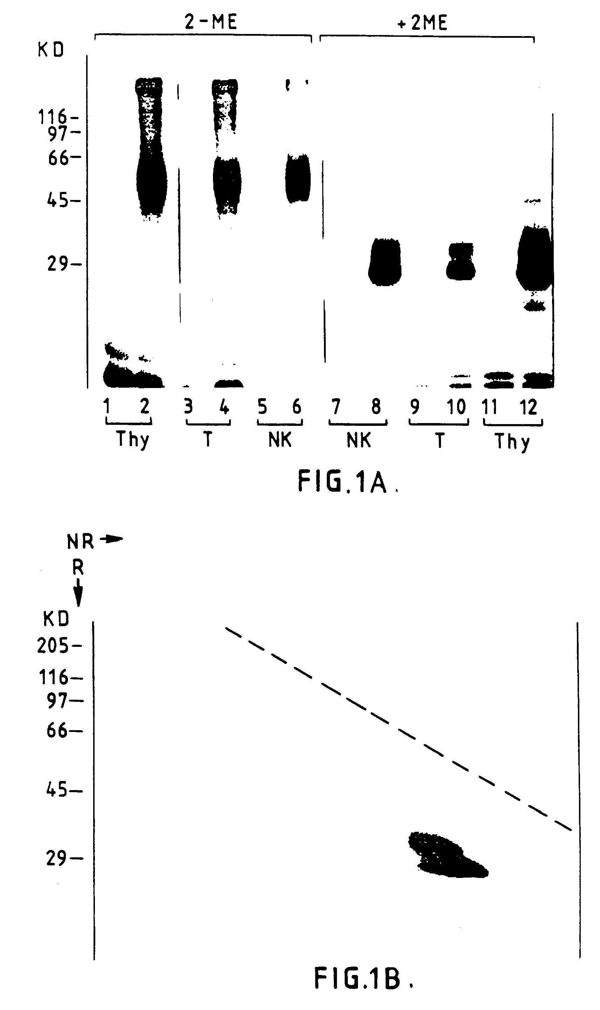

As shown in Fig. 1A, an essentially identical structure was immunoprecipitated from IL-2 dependent cell lines of NK cells, T lymphocytes and thymocytes. Anti-Leu 23 immunoprecipitated a disulfide-bonded 50-60 kD glycoprotein that dissociated into subunits of 32 kD and 28 kD after reduction. In Fig. 1B, two-dimensional "diagonal" SDS-PAGE analysis confirmed disulfide-bonding of the subunits, and the relative mobility of the subunits below the diagonal suggested that these proteins may form dimers of 32 kD + 32 kD, 32 kD + 28 kD, and 28 kD + 28 kD proteins.

Further analyses revealed the extent of glycosylation. Leu 23 was eluted from the S. aureus/immune complex previously described by adding 20 ul of phosphate buffer (0.1M, pH 6.1) containing 50 mM EDTA, 1% 2-ME and 1% SDS. After incubation for 20 minutes, the complex was centrifuged, the eluate removed and the pellet diluted ten-fold in phosphate buffer containing 50 mM EDTA and 1% NP-40. 25ul aliquots were placed in microfuge tubes. Endo-F (New England Nuclear) was added to a final concentration of 23 U/ml and the samples were incubated for 16 hours at 37°C. An equal volume of 2X sample buffer containing 10% 2-ME then was added to stop digestion and the tubes placed in a boiling water bath for 5 minutes. SDS-PAGE analysis then was performed.

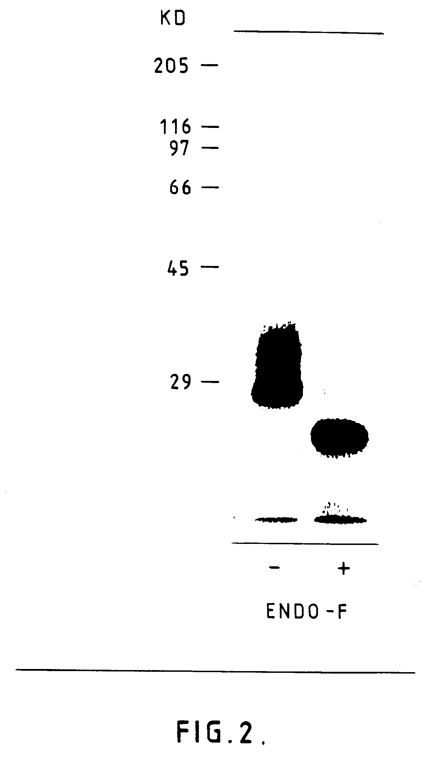

Deglycosylation of the antigen with a pre-determined optimal concentration of Endo F enzyme revealed a single protein of 24 kD. See Fig. 2. A partially deglycosylated species was observed with a relative mobility of 28 kD. Since Endo F cleaves both high mannose and complex types of carbohydrates linked through asparagine to the peptide, the Leu 23 antigen is apparently a protein of 24 kD with at least 2 sites for N-linked glycosylation. Two-dimensional NEPHGE analysis of the intact and deglycosylated Leu 23 antigen further indicated that the 32 kD and 28 kD subunits likely represent the same polypeptide that differ by the addition of a single N-linked carbohydrate. The deglycosylated 24 kD protein was resistant to further digestion with N-glycanase, an enzyme that hydrolyzes asparagine-linked oligosaccharides that may be resistant to Endo F treatment. The microheterogeneity observed in the deglycosylated 24 kD protein may have resulted from carbamylation of the protein by urea during NEPHGE analysis, or alternatively could be due to the presence of enzyme-resistant carbohydrates or 0-linked oligosaccharides.

Apart from the structural characteristics of Leu 23, the antigen appears to be uniquely regulated and provides a method to monitor/detect activation or stimulation. The conditions necessary to induce expression of Leu 23 were defined.

LBD and HBD were isolated as previously described in J. H. Phillips, N.L. Warner and L. L. Lanier, Nat. Immun. Cell Growth Regul., 3:73 (1984). Lymphocytes were cultured in 500 U/ml rIL-2 and aliquots were removed after 2, 6, and 18 hours of culture. Cells were stained by two-color immunofluorescence with PE-conjugated Anti-Leu 23 and either FITC-conjugated Anti-Leu 11 (CD16) to identify NK cells or FITC-conjugated Anti-Leu 4 (CD3) to identify T lymphocytes.

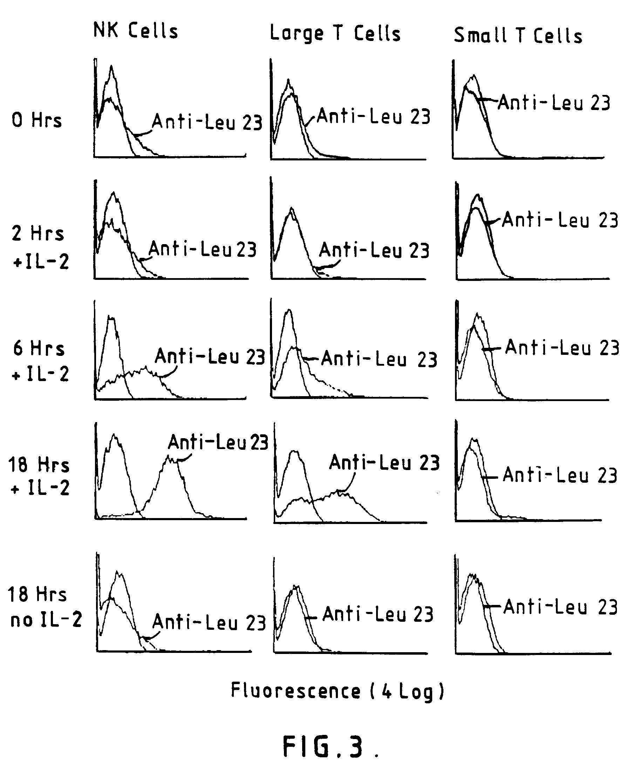

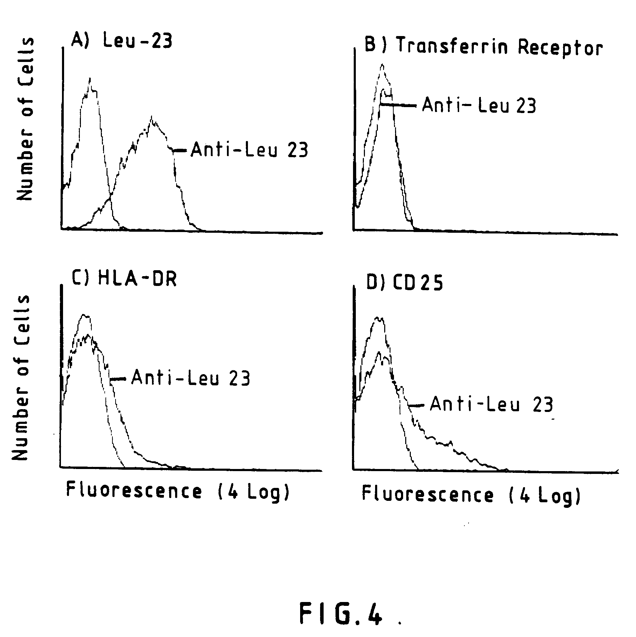

Prior to stimulation with rIL-2, Leu 23 was not detectable on LBD (large) T cells or HBD (small) T cells, and was present only in low amounts on a minor proportion of NK cells. See Fig. 3. Leu 23 was detected, however, by 6 hours after rIL-2 stimulation on the majority of NK cells and a subset of the T lymphocytes in the LBD fraction. Within 18 hours, essentially all rIL-2 stimulated NK cells expressed Leu 23. By contrast, only a minor proportion of rIL-2 stimulated NK cells expressed transferrin receptors, HLA-DR or CD25. See Fig. 4. Since all NK cells expressed Leu 23 within 18 hours after exposure to rIL-2, this indicates that all NK cells are IL-2 responsive. NK cells maintained in culture with rIL-2 for 72 hours remained uniformly Leu 23 positive. Whereas all NK cells acquired Leu 23, even after 72 hours of culture with rIL-2 only a subset of the T lymphocytes in the LBD fraction expressed this antigen. In contrast to the T lymphocytes in the LBD fraction, rIL-2 did not induce Leu 23 expression on a substantial proportion of the small, resting T lymphocytes in the HBD (Fig. 3), even after 72 hours of culture.

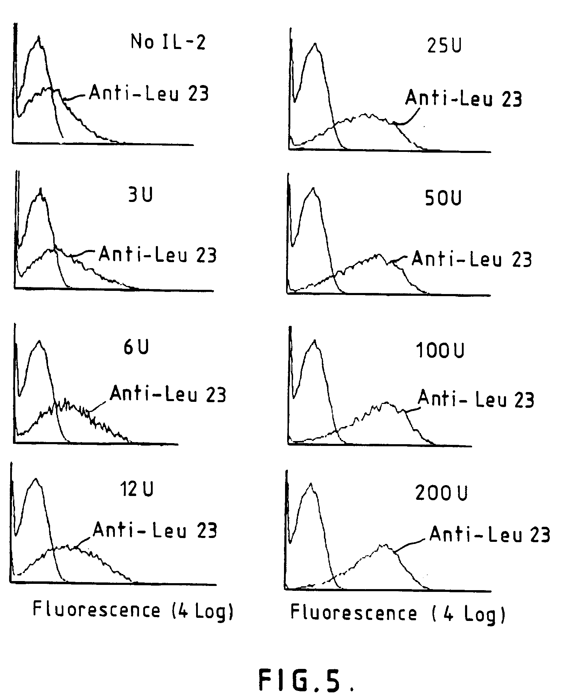

Induction of Leu 23 antigen by IL-2 was dose dependent. Referring to Fig. 5, after 18 hours of culture, low levels of Leu 23 antigen were induced on NK cells at 3 U/ml rIL-2 and 100 U/ml rIL-2 was sufficient for induction of the antigen on a majority of NK cells. IL-2 was necessary and sufficient to induce expression of Leu 23 on NK cells. NK cells, purified by isolating CD16+ lymphocytes from the LBD fraction using FACS440™ cell sorting instrumentation acquired expression of Leu 23 after 18 hour culture with rIL-2. T lymphocytes, monocytes, or other accessory cells were not required for IL-2 to induce the expression of Leu 23 on NK cells.

Further studies were conducted to determine the correlation of IL-2 enhanced cytotoxicity with expression of Leu 23. LBD cells were cultured in serum free media with 100 U/ml rIL-2 for 18 hours in the presence or absence of neutralizing rabbit or goat anti-IL-2 serum.

Goat anti-IL-2 was used at a dilution to completely neutralize 100 U/ml rIL-2. Rabbit anti-IL-2 (Genzyme) was used at a dilution to achieve 50% neutralization of 100 U/ml rIL-2. After 18 hours, cells were harvested and analyzed for 4 hours for cytotoxicity against 51Cr (Amersham) labeled tumor target cells (i.e., Colo-205, ATCC No. CCL 222) at an effector to target ratio of 12:1. These cells were further analyzed by flow cytometry after staining with FITC-conjugated control IgG or Anti-Leu 23 (FITC).

As shown in Table II, both IL-2 enhanced cytotoxicity against a colon carcinoma cell line and induction of Leu 23 on NK cells were comparably inhibited by the neutralizing antiserum. There is a quantitative relationship between inhibition of cytotoxicity and inhibition of Leu 23 antigen induction. These results demonstrate that both phenomenon are IL-2 dependent, and possibly co-regulated events.

TABLE II

| Antiserum | IL-2 | % Cytotoxicity | | % Inhibition Colo-205 Lysis | % Inhibition Leu-23 Antigen |
|---|---|---|---|---|---|
| | | K562 | Colo-205 | | |
| None | - | 43 | 0 | | |
| None | + | 97 | 60 | | |
| Normal Serum | + | 100 | 65 | 0 | 0 |
| Rabbit anti-IL-2 | + | 69 | 20 | 69 | 50 |
| Goat anti-IL-2 | + | 41 | 0 | 100 | 96 |

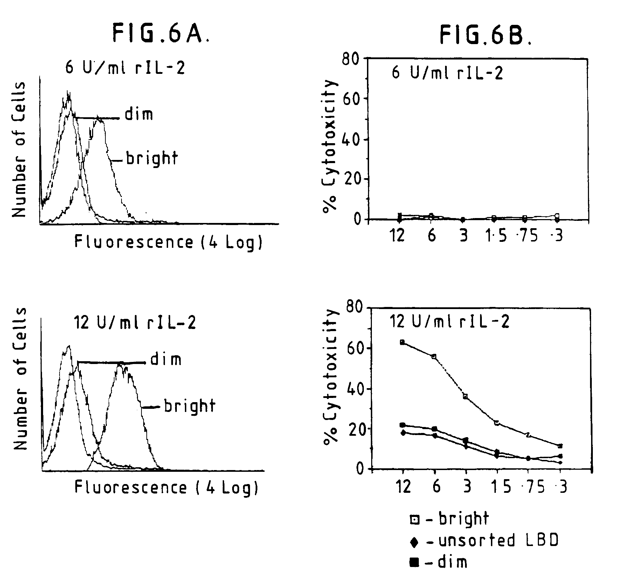

Referring to Fig. 6A, experiments were performed to determine whether the level of Leu 23 antigen expression would correlate with lytic activity. LBD cells were cultured with 6 U/ml and 12 U/ml rIL-2 for 18 hours and then were stained with FITC-conjugated Anti-Leu 11 and PE conjugated Anti-Leu 23. Low levels of Leu 23 were induced on the CD16$^+$ NK cell population under these conditions. Although no distinct negative or positive subsets of Leu 23 were evident, it was possible to identify CD16$^+$ NK cells that were negative or expressed low (or "dim") amounts of Leu 23 antigen and CD16$^+$ NK cells that expressed relatively high (or "bright") levels of Leu 23 antigen.

Referring to Fig. 6B, using 6 U/ml rIL-2 for induction, no augmentation of cytotoxicity was observed against an NK-insensitive colon carcinoma cell line (Colo-205), even though low levels of Leu 23 antigen could be detected on the NK cell population. These results indicate that Leu 23 antigen can be expressed prior to detection of cytotoxic activity. Using 12 U/ml rIL-2 for induction, cytotoxicity was mediated by NK cells that expressed both "dim" and "bright" levels of Leu 23 antigen. Comparison of the cytotoxic activity mediated at several effector to target ratios clearly indicated that NK cells expressing higher levels of Leu 23 antigen were more lytic than the population of NK cells lacking Leu 23 or expressing low levels of this antigen.

The correlation of cytotoxic of lytic activity with the expression of Leu 23 within hours after cell activation by IL-2 provides a means to monitor cytotoxic function without the necessity of waiting for the expression of the transferrin or IL-2 receptors. This then provides the clinician with a means to confirm cell activation prior to administration which may result in improved treatment.

For example, in the method for LAK therapy proposed by Rosenberg, see U.S. Pat. No. 4,690,915, which is incorporated herein by reference, autologous LAK cells were isolated and combined with rIL-2 for 3 to 4 days prior to I/V administration. Cytotoxic activity was measured by the ability of the LAK cells to lyse NK-insensitive tumor cell lines.

As a replacement for this lengthy procedure, a sample of the rIL-2 activated cells are combined with Anti-Leu 23. The staining of rIL-2 activated cells may be detected by direct fluorescence (e.g., coupling Anti-Leu 23 to a fluorochrome such as FITC or PE, a phycobiliprotein) or by indirect fluorescence (e.g., using FITC-conjugated goat anti-mouse Ig). In either event, the degree of staining of activated cells is correlated with cytotoxicity. Thus, the cells from which the sample is taken may be administered for treatment knowing that the cells have been effectively activated.

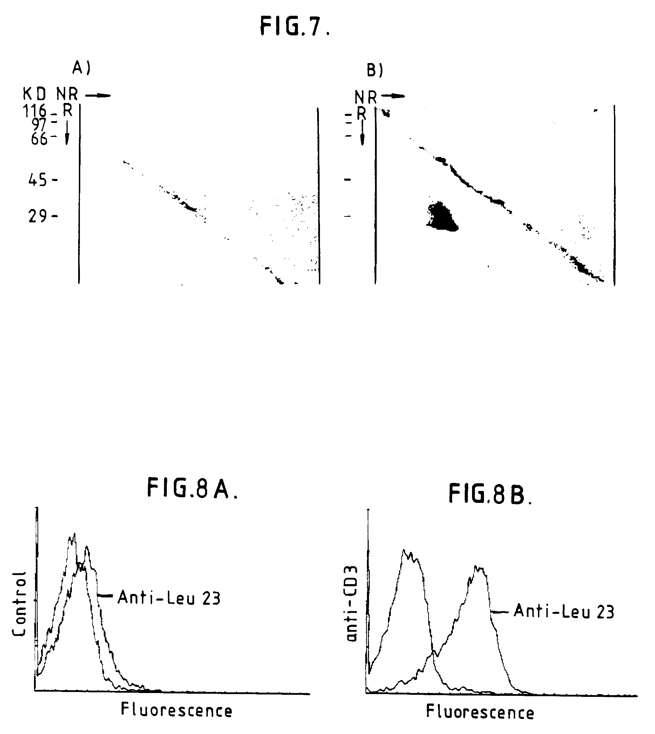

Similarly, the correlation of mitogen stimulation of the T cell antigen receptor with the expression of Leu 23 provides an additional means to monitor leukocyte activation. Referring to Fig. 8, the T leukemia cell line, Jurkat, express Leu 23 after overnight stimulation with Anti-Leu 4. As such, physological stimulation of normal T cells via the T cell antigen receptor with specific antigens (e.g., alloantigen, virus or autoantigen) may result in expression of Leu 23. Anti-Leu 23, therefore, may be combined with leukocytes from a patient or person thought to be expressing indications of viral infection, for example. By using direct or indirect immunofluorescence with Anti-Leu 23, Leu 23 may be detected. Treatment then may be appropriately prescribed.

Finally, it was found that Leu 23 is both induced and phosphorylated as a consequence of IL-2 stimulation. LBD cells were washed and incubated for 1 hour at 37°C in phosphate-free buffer (modified MEM phosphate-free Earle's salts) with 25mM HEPES (Irvine Scientific) supplemented with 1mM glutamine (GIBCO) non-essential amino acids, 100 ug/ml gentamycin (GIBCO) and 2% heat inactivated horse serum (KC Biologicals). Cells were resuspended in the phosphate-free buffer containing 1.25 mCi/ml [$^{32}$P]-orthophosphate (carrier-free, Amersham Corp.) and 800 U/ml rIL-2. Cells were incubated for 3 hours at 37°C and then washed three times in cold PBS containing 0.1 mM $Na_3VO_4$, 0.4 mM EDTA, 10 mM $Na_4P_2O_7$, 10 mM NaF and 0.1% $NaN_3$. Cells then were solubilized, lysates and eluates prepared as previously described.

Referring to Fig. 7, disulfide-bonded [$^{32}$P]-labeled protein dimers composed of 32 kD and 28 kD subunits were detected. These subunits were identical to those presented in Fig. 1B. The induction and phosphorylation of Leu 23, therefore, is similar to the results seen with lymphocyte stimulation which results in activation of intracellular protein kinases which in turn phosphorylate growth factor receptors, components of T cell antigen receptor complex and other membrane glycoproteins.

Thus, Anti-Leu 23 identifies an antigen which is rapidly induced on peripheral blood NK cells and a unique subset of T lymphocytes upon exposure to IL-2, without the necessity of exogenous co-factors or accessory cells. The kinetics of IL-2 induced expression of Leu 23 on NK cells closely parallels the acquisition of cytotoxic function against solid tumor targets, thereby providing a useful marker for NK cell activation.

## Claims

1. A hybridoma which produces a monoclonal antibody, said monoclonal antibody interacting with the cellular antigen which is recognised by the monoclonal antibody produced by the hybridoma designated as HB-9627.

2. A monoclonal antibody interacting with the cellular antigen which is recognised by the monoclonal antibody produced by the hybridoma designated as ATCC No. HB-9627.

3. A method for monitoring activation of leukocytes in a biological sample which comprises combining said sample with a monoclonal antibody interacting with the cellular antigen which is recognised by the monoclonal antibody produced by the hybridoma designated as HB-9627, detecting the combination by direct or indirect fluorescence means and detecting fluorescence by flow cytometry or fluorescence microscopy means.

4. A method according to Claim 3 wherein the activated leukocytes are selected from the group consisting of T lymphocytes, B lymphocytes and NK cells.

5. A method according to Claim 4 wherein the activated leukocytes are NK cells.

6. A method according to Claim 4 wherein the activated leukocytes are T lymphocytes.

7. A method according to any of Claims 3 to 6 wherein the monoclonal antibody is the monoclonal antibody produced by the hybridoma designated as ATCC No. HB-9627.

8. A method according to any of Claims 3 to 7 wherein the monoclonal antibody is directly coupled to a fluorochrome.

9. A method according to Claim 8 wherein the fluorochrome is selected from the group consisting of fluorescein isothiocyanate and phycobiliprotein.

10. A method according to any of Claims 3 to 9 wherein the leukocytes in the sample are activated by IL-2 or are activated via the T cell antigen receptor by a foreign antigen, autoantigen, anti-T cell receptor antibody or anti-CD3 antibody.

## Patentansprüche

1. Hybridom, welches einen monoklonalen Antikörper produziert, wobei der monoklonale Antikörper mit dem Zell-Antigen in Wechselwirkung tritt, welches durch den monoklonalen Antikörper erkannt wird, der durch das als HB-9627 bezeichnete Hybridom produziert wird.

**2.** Monoklonaler Antikörper, der mit dem Zell-Antigen in Wechselwirkung tritt, welches durch den monoklonalen Antikörper erkannt wird, der durch das als ATCC Nr. HB-9627 bezeichnete Hybridom produziert wird.

**3.** Methode zur Überwachung der Aktivierung von Leukozyten in einer biologischen Probe, welche die Kombination der Probe mit einem monoklonalen Antikörper, der mit dem Zell-Antigen in Wechselwirkung tritt, welches durch den monoklonalen Antikörper erkannt wird, der durch das als HB-96627 bezeichnete Hybridom produziert wird, den Nachweis der Kombination durch Methoden der direkten oder indirekten Fluoreszenz und die Feststellung der Fluoreszenz durch Methoden der Strömungszytometrie oder der Fluoreszenzmikroskopie umfaßt.

**4.** Methode nach Anspruch 3, bei welcher die aktivierten Leukozyten aus der Gruppe ausgewählt werden, die aus T-Lymphozyten, B-Lymphozyten und NK-Zellen besteht.

**5.** Methode nach Anspruch 4, bei welcher die aktivierten Leukozyten NK-Zellen sind.

**6.** Methode nach Anspruch 4, bei welcher die aktivierten Leukozyten T-Lymphozyten sind.

**7.** Methode nach einem der Ansprüche 3 bis 6, bei welcher der monoklonale Antikörper der monoklonale Antikörper ist, der durch das als ATCC Nr. HB-9627 bezeichnete Hybridom produziert wird.

**8.** Methode nach einem der Ansprüche 3 bis 7, bei welcher der monoklonale Antikörper direkt an ein Fluorochrom gekoppelt ist.

**9.** Methode nach Anspruch 8, bei welcher das Fluorochrom aus der Gruppe ausgewählt wird, die aus Fluoreszein-Isothiocyanat und Phykobiliprotein besteht.

**10.** Methode nach einem der Ansprüche 3 bis 9, bei welcher die Leukozyten in der Probe durch IL-2 aktiviert werden oder über den T-Zellen-Antigen-Rezeptor durch ein Fremdantigen, Autoantigen, einen Anti-T-Zellen-Rezeptor-Antikörperoder einen Anti-CD3-Antikörper aktiviert werden.

**Revendications**

**1.** Hybridome qui produit un anticorps monoclonal, ledit anticorps monoclonal interagissant avec l'antigène cellulaire qui est reconnu par l'anticorps monoclonal produit par l'hybridome désigné comme HB-9627.

**2.** Anticorps monoclonal interagissant avec l'antigène cellulaire qui est reconnu par l'anticorps monoclonal produit par l'hybridome désigné comme ATCC No HB-9627.

**3.** Procédé pour contrôler l'activation de leucocytes dans un échantillon biologique qui comprend la combinaison dudit échantillon avec un anticorps monoclonal interagissant avec l'antigène cellulaire qui est reconnu par l'anticorps monoclonal produit par l'hybridome désigné comme HB-9627, la détection de la combinaison par un moyen de fluorescence directe ou indirecte et la détection de la fluorescence par cytométrie en écoulement ou par un moyen de microscopie de fluorescence.

**4.** Procédé selon la revendication 3, dans lequel les leucocytes activés sont choisis dans le groupe constitué par les lymphocytes T, les lymphocytes B et les cellules NK.

**5.** Procédé selon la revendication 4, dans lequel les leucocytes activés sont des cellules NK.

**6.** Procédé selon la revendication 4, dans lequel les leucocytes activés sont des lymphocytes T.

**7.** Procédé selon l'une quelconque des revendications 3 à 6, dans lequel l'anticorps monoclonal est l'anticorps monoclonal produit par l'hybridome désigné comme ATCC No HB-9627.

**8.** Procédé selon l'une quelconque des revendications 3 à 7, dans lequel l'anticorps monoclonal est directement couplé à un fluorochrome.

**9.** Procédé selon la revendication 8, dans lequel le fluorochrome est choisi dans le groupe constitué par l'isothiocyanate de fluorescéine et la phycobiliprotéine.

**10.** Procédé selon l'une quelconque des revendication 3 à 9, dans lequel les leucocytes dans l'échantillon sont activés par IL-2 ou sont inactivés via le récepteur de l'antigène de cellule T par un antigène étranger, un autoantigène, un anticorps récepteur de cellule anti-T ou un anticorps anti-CD3.

FIG.1A.

FIG.1B.

FIG.2.

Fluorescence ( 4 Log )

FIG.3.

FIG.4 .

FIG.5.

FIG.6A.

FIG.6B.

## FIG.7.

## FIG.8A.

## FIG.8B.